# EUROPÄISCHE PATENTANMELDUNG

(11) **EP 2 626 046 A1**
(43) Veröffentlichungstag der Anmeldung: **14.08.2013**
(21) Anmeldenummer: 13150460.7
(22) Anmeldetag: 08.01.2013
(51) Int. Cl.: A61F 5/01

(54) **Orthese**

(30) Priorität: 09.02.2012 DE 102012002552
(71) Anmelder: Pohlig GmbH, 83278 Traunstein (DE)
(72) Erfinder: Pohlig, Kurt, 83278 Traunstein (DE); Schäfer, Michael, 83278 Traunstein (DE); Starker, Felix, 70563 Stuttgart (DE)
(74) Vertreter: Pfenning, Meinig & Partner GbR

(57) **Zusammenfassung**

Orthese zur Korrektur von Gelenkfehlstellungen mit einem ersten Schalenteil (1) zur Aufnahme eines ersten Körperteils, einem zweiten Schalenteil (2) zur Aufnahme eines zweiten mit dem ersten Körperteil durch ein Gelenk verbundenen zweiten Körperteils und mindestens einem Orthesengelenk (3) mit zwei gegeneinander verdrehbaren Gelenkstücken, wobei ein erstes Gelenkstück (4) des Orthesengelenks an mindestens drei Befestigungspunkten (9) auf einer durch die Befestigungspunkte definierten Befestigungsfläche (10) des ersten Schalenteils um einen Drehpunkt (5) des Orthesengelenks herum derart befestigt ist, dass der Drehpunkt des Orthesengelenks senkrecht beabstandet zur Befestigungsfläche liegt, und dass die Orthese eine Schiene (6) mit einem ersten und einem zweiten Teil aufweist, deren erster Teil als zweites Gelenkstück (7) des Orthesengelenks ausgebildet ist und deren zweiter Teil am zweiten Schalenteil befestigt ist.

## Beschreibung

Die Erfindung betrifft eine Orthese zur Korrektur von Gelenkfehlstellungen.

Derartige Orthesen weisen üblicherweise ein erstes und ein zweites Schalenteil sowie ein Orthesengelenk auf. Die Schalenteile dienen zur Aufnahme von Körperteilen, welche durch ein körperliches Gelenk miteinander verbunden sind. Durch das Tragen einer Orthese wird der Bewegungsbereich des betreffenden Gelenks auf einen fest vorgegebenen Bewegungsbereich eingeschränkt, so dass eine Bewegung des Gelenks nur entlang vorgegebener Freiheitsgrade ermöglicht wird. Orthesen werden zur konservativen Therapie eines Gelenks als auch nach Gelenkoperationen eingesetzt, um postoperative Gelenkeinsteifungen oder einen Rückfall in eine bereits durch Operation korrigierte Gelenkfehlstellung zu vermeiden.

Aus DE 199 28 269 B4 ist bereits eine Beinorthese mit einem Universalgelenk bekannt, welches ein erstes Schalenteil zur Aufnahme eines Fußes und ein zweites Schalenteil zur Aufnahme eines Unterschenkels aufweist. Das beschriebene Universalgelenk besteht im Wesentlichen aus einem Kugelgelenklager, welches zwei gegeneinander bewegliche Gelenkteile miteinander verbindet. An einem ihrer Enden sind die Gelenkteile jeweils mit einem Schalenteil verbunden. Dies ermöglicht Schwenkbewegungen der Beinorthese, wobei der Drehpunkt des Orthesengelenks mittig zwischen dem ersten und dem zweiten Schalenteil liegt. Am zweiten Schalenteil befindet sich ferner oberhalb des Orthesengelenks ein Anschlagblock. Dieser beschränkt die Bewegung des mit dem ersten Schalenteil fest verbundenen Gelenkteils parallel sowie senkrecht zur Orthese.

Nachteilig an dieser Gelenkvariante ist zum einen, dass zwei längliche Gelenkteile bzw. Schienen und ein Anschlagblock, welcher die Bewegung parallalel und senkrecht zur Orthese beschränkt, für die Funktion des Orthesengelenks notwendig sind. Hierdurch ist eine dreidimensionale Bewegung des Sprunggelenks kaum möglich. Zum anderen führt die Verwendung von derart vielen und großen Gelenksbestandteilen zur Erhöhung des Gesamtgewichts der Orthese, wodurch Beweglichkeit und Komfort des Patienten stark einschränkt werden.

Die US 7,044,926 B2 beschreibt ebenfalls eine Beinorthese mit zwei Schalenteilen, jeweils zur Aufnahme eines Fußes und eines Unterschenkels, und einem kugelgelagerten Orthesengelenk. Das kugelgelagerte Orthesengelenk wird durch zwei Gelenkkomponenten zusammengehalten, wobei jeweils ein Ende einer Gelenkkomponente mit dem Fußabschnitt bzw. dem Beinabschnitt der Orthese verbunden ist. Der Drehpunkt des Kugelgelenks befindet sich somit zwischen dem Fuß- und dem Beinabschnitt und ermöglicht eine dreidimensionale Bewegung des Sprunggelenks. Dadurch passt sich die Orthese einer Fehlstellung eines Gelenks bis zu einem gewissen Grad an, wodurch das Anlegen der Orthese erleichtert wird.

Nachteilig an dieser Gelenkvariante ist jedoch ebenfalls die Verwendung von großen, die Optik der Orthese dominierenden Gelenkkomponenten, wodurch sich zum einen das Gesamtgewicht der Orthese erhöht und zum anderen die allgemeine Optik der Orthese beeinträchtigt wird.

Die Aufgabe der vorliegenden Erfindung ist es daher, eine Orthese bereitzustellen, welche einen gegebenenfalls großen dreidimensionalen Bewegungsbereich des Orthesengelenks ermöglicht bei gleichzeitiger Feineinstellbarkeit des Bewegungsbereichs des Orthesengelenks. Zusätzlich ist es Aufgabe der vorliegenden Erfindung, die Orthese in ihrem Gesamtgewicht zu reduzieren und mit einer ansprechenden Optik zu versehen.

Diese Aufgabe wird durch die Orthese zur Korrektur von Gelenkfehlstellungen nach Anspruch 1 gelöst. Vorteilhafte Weiterbildungen der erfindungsgemäßen Orthese sind in den abhängigen Ansprüchen 2 bis 11 beschrieben.

Die vorliegende Erfindung ermöglicht eine erweiterte dreidimensionale Beweglichkeit des Körpergelenks innerhalb eines vorgegebenen Bewegungsbereichs, welcher sich individuell jederzeit einstellen lässt. Hierdurch wird eine gesteigerte Heilungsrate erreicht.

Ferner ist die erfindungsgemäße Orthese leicht, optisch ansprechend und somit komfortabler als die im Stand der Technik bekannten Orthesen. Dies fördert wiederum die Bereitwilligkeit des Patienten, die Orthese häufiger anzuwenden, und zwar nicht nur zu Hause, sondern auch im Alltag, und somit auch eine Steigerung der Heilungsrate.

Die erfindungsgemäße Orthese zur Korrektur von Gelenkfehlstellungen weist einen ersten Schalenteil zur Aufnahme eines ersten Körperteils sowie einen zweiten Schalenteil zur Aufnahme eines zweiten Körperteils auf. Das erste und das zweite Körperteil sind dabei durch ein körperliches Gelenk miteinander verbunden. Außerdem weist die Orthese mindestens ein Orthesengelenk mit zwei gegeneinander verdrehbaren Gelenkstücken auf. Das erste Gelenkstück des Orthesengelenks ist an mindestens drei Befestigungspunkten auf einer durch die Befestigungspunkte definierten Befestigungsfläche des ersten Schalenteils um einen Drehpunkt des Orthesengelenks herum befestigt. Die Befestigungsfläche ist somit Teil der von der Orthese weg gerichteten Außenfläche des ersten Schalenteils. Das erste Gelenkstück ist nun derart befestigt, dass der Drehpunkt des Orthesengelenks senkrecht zur Befestigungsfläche beabstandet liegt. Die Orthese weist ferner eine Schiene mit einem ersten und einem zweiten Teil auf. Dabei ist der erste Teil der Schiene als zweites Gelenkstück des Orthesengelenks ausgebildet, und der zweite Teil der Schiene am zweiten Schalenteil befestigt.

Das erste Gelenkstück kann dabei in dem ersten Schalenteil verankert, an dem ersten Schalenteil angeschraubt, angenietet oder in das erste Schalenteil einlaminiert sein.

Die erfindungsgemäße Orthese kann als monolaterale Orthese ausgebildet sein. In diesem Fall weist die Orthese lediglich ein einzelnes Orthesengelenk auf. Bei einer derartigen monolateralen Orthese ist gegebenenfalls ein größerer dreidimensionaler Bewegungsbereich und somit eine individuellere Einstellbarkeit des Bewegungsbereichs des Orthesengelenks möglich. Dies steigert die Heilungsrate. Ferner ist das Anlegen der Orthese mit monolateralem Orthesengelenk bei einem Gelenk mit Fehlstellung einfacher.

Alternativ kann die Orthese auch als bilaterale Orthese ausgebildet sein. In diesem Fall weist die Orthese zwei bilateral angeordnete Orthesengelenke auf. Der Vorteil einer bilateralen Orthese ist die erhöhte Belastbarkeit und Stabilität der Orthese, welche dadurch länger verwendbar und auch für schwerere Patienten anwendbar ist.

Die erfindungsgemäße Orthese kann wahlweise mit einem Kugel- oder einem Scharniergelenk ausgestattet sein. Ein Kugelgelenk ermöglicht eine dreidimensionale Bewegung des körperlichen Gelenks und somit eine individuellere Einstellbarkeit des Bewegungsbereichs des Orthesengelenks. Bei der Verwendung eines Scharniergelenks kann die Orthese stärker belastet werden und hat eine höhere Haltbarkeit.

Für den Fall, dass das Orthesengelenk ein Kugelgelenk ist, weist das Orthesengelenk zusätzlich zum ersten und zweiten Gelenkstück eine erste Anschlagscheibe zum Einschränken einer Bewegung des die Körperteile verbindenden Gelenks in eine erste Richtung, einen Innenring mit einer sphärisch und konvex gekrümmten Oberfläche und einer Abdeckscheibe auf. Dabei ist das erste Gelenkstück als kreisförmige Scheibe mit einer senkrecht und zentral auf der Scheibe stehenden Gelenkachse ausgebildet, die erste Anschlagscheibe auf die Gelenkachse und dahinter der Innenring aufgeschoben, auf dem Innenring das zweite Gelenkstück gelagert und die Abdeckscheibe zum Verschließen und/oder Öffnen des Orthesengelenks und/oder zur Sicherung der Gelenkachse an einem dem ersten Gelenkstück gegenüberliegenden Ende der Gelenkachse angeordnet.

Vorteilhafterweise weist das Orthesengelenk zwischen dem Innenring und der ersten Anschlagscheibe und/oder zwischen dem Innenring und der Abdeckscheibe eine zweite Anschlagscheibe zum Einschränken der Bewegung des die Körperteile verbindenden Gelenks in eine zweite Richtung auf.

Der Innenring enthält und/oder besteht vorzugsweise vollständig aus Teflon.

Die erste und/oder die zweite Anschlagscheibe des Orthesengelenks können derart ausgebildet sein, dass sie mittels Madenschrauben bzw. Gewindestiften und/oder Schrauben oder/und mittels sich auf dem ersten Gelenkstück sowie auf den Anschlagscheiben befindlichen Rasten einstellbar sind. Vorzugsweise ist das Einstellen der Anschlagscheiben in Schritten von 2° oder von 5° oder von Vielfachen des Abstands der Madenschrauben bzw. Gewindestiften und/oder Schrauben zueinander möglich.

Vorzugsweise weist das zweite Gelenkstück zusätzlich Mittel zum Beschränken des Bewegungsbereichs des die Körperteile verbindenden Gelenks bzw. des Orthesengelenks in einer Richtung parallel zur Gelenkachse auf. Derartige Mittel wären beispielsweise Gewindestifte, die konzentrisch um das Kugelgelenklager in das zweite Gelenkstück eingeschraubt sind und deren Länge größer ist als die Dicke des ringförmigen zweiten Gelenkstücks, gemessen in einer Richtung parallel zur Gelenkachse. Die Länge der überstehenden Enden der Gewindestifte bestimmt den Abstand zu einer bzw. zu beiden benachbarten Anschlagscheiben und somit den Neigungswinkel des zweiten Gelenkstücks innerhalb des Orthesengelenks.

Desweiteren kann die Orthese derart ausgebildet sein, dass die Position der Befestigungsfläche auf dem ersten Schalenteil variierbar ist und dadurch die Position des Drehpunkts des Orthesengelenks individuell positionierbar ist. Hierdurch lässt sich ebenfalls der von der Orthese aufgezwungene Bewegungsbereich des Körpergelenks individuell an den Patienten und die Gelenkfehlstellung anpassen.

Ferner ist es möglich, dass die Schalenteile der Orthese durch Entfernen des ersten Gelenkstücks vom zweiten Gelenkstück voneinander separierbar sind. Vorteilhafterweise sind das erste und das zweite Gelenkstück mittels eines Klippmechanismus oder/und Druckknopfmechanismus voneinander entfernbar oder miteinander verbindbar. Dies ermöglicht jederzeit ein einfaches und schnelles Anlegen bzw. Ablegen der Orthese.

Die Orthese kann auch derart ausgebildet sein, dass sich das zweite Gelenkstück am Ende der Schiene befindet. Vorteilhaft hieran ist, dass die Schiene kleiner gebaut und somit Material eingespart werden kann, wodurch sich das Gesamtgewicht der Orthese verringert und die Optik der Orthese verbessert wird.

Eine weitere Ausführungsform der Orthese sieht vor, dass das erste Schalenteil eine Ringfassung aufweist, z.B. zur Aufnahme eines Fußes. Dies verbessert den Sitz der Orthese am Fuß und macht ein zusätzliches Verschlusssystem im Spannbereich überflüssig.

Zum Reduzieren des Gewichts der Orthese können Ausnehmungen in die Schalenteile eingebracht werden. Neben der Gewichtsreduktion wird hierdurch eine bessere Transpiration erreicht.

Ferner kann die Schiene der Orthese und/oder das Orthesengelenk teilweise oder vollständig aus Metall, Leichtmetall, insbesondere Aluminium, Magnesium oder/und Edelstahl, Kunststoff, einem Kunststoffmetallverbund oder einem Verbund mehrerer oder aller vorstehend genannten Materialien bestehen. Die Schalenteile der Orthese bestehen vorzugsweise teilweise oder vollständig aus einem Glasfaserverbund, Kohlefaserverbund, Basaltfaserverbund oder einem Verbund mehrerer oder aller vorstehend genannter Materialien. Die Verwendung eines Basaltfaserverbunds ist dabei besonders vorteilhaft, da dieser im Vergleich zu einem Glasfaserverbund eine höhere Zug- und Biegefestigkeit aufweist und gleichzeitig im Preis günstiger ist.

Die Orthese kann derart ausgebildet sein, dass das erste und/oder das zweite Schalenteil zum Fixieren des ersten bzw. zweiten Körperteils mindestens eine Rastenschnalle, einen Ratschenverschluss, einen Rastenklettverschluss oder ein metallisches Klettverschlusssystem aufweisen. Ein metallischer Klettverschluss ist mechanisch hoch belastbar bei einer zugleich minimalen Bauhöhe der Komponentenstrukturen.

Auf die Schalenteile ist vorzugsweise eine Deckschicht mit verschiedenen Farb- und/oder Mustervarianten auftragbar. Alternativ können die Schalenteile mittels Wassertransferdruck beschichtet werden.

Die erfindungsgemäße Orthese kann u.a. als Sprunggelenksorthese ausgebildet sein. Dabei ist das erste Schalenteil zur Aufnahme eines Fußes und das zweite Schalenteil zur Aufnahme eines Unterschenkels vorgesehen. Vorteilhafterweise weist das erste Schalenteil eine gegebenfalls öffenbare Fersenkappe zur Integration der Ferse des Fußes und zur zuverlässigen Verriegelung der Ferse im Fußteil auf.

Die Fersenkappe kann mit einem metallischen Klettverschluss, einem Rastenklettverschluss, einem Schwalbenschwanzverschluss, einem Ratschenverschluss, einem Kunststoffklettbandverschluss oder einem Seilzugsystem verstellbar, insbesondere öffenbar und/oder verschließbar sein. Vorteilhaft an einem Rastenklettverschluss ist die zusätzliche Führung der Verschlusskomponenten orthogonal zur Zugrichtung bei einer zugleich hochfesten Verbindung und äußerst flacher Bauweise.

Für den Fall, dass die Fersenkappe mit einem metallischen Klettverschluss verschließbar, insbesondere öffenbar und/oder verschließbar ist, kann der metallische Klettverschluss metallische Federstahlbleche mit ausgestanzten Haken und Federstahlbleche mit eingeprägten Ösen aufweisen. Beim Ineinandergreifen haften die Haken und Ösen aneinander.

Für den Fall, dass die Orthese als Sprunggelenksorthese ausgebildet ist, kann die Orthese derart ausgebildet sein, dass das Orthesengelenk oder/und das Sprunggelenk bei angelegter Orthese seitlich um maximal 12° kippbar ist. Ferner können in diesem Fall die Anschlagscheiben derart einstellbar sein, dass das Sprunggelenk um 30° zu einer horizontalen Ebene nach unten abwinkelbar und um 40° zur horizontalen Ebene nach oben anwinkelbar ist.

Weiterhin kann die erfindungsgemäße Orthese dadurch gekennzeichnet sein, dass die Schalenteile durch Entfernen des ersten Gelenkstücks vom zweiten Gelenkstück voneinander separierbar sind.

Weiterhin kann die erfindungsgemäße Orthese dadurch gekennzeichnet sein, dass das erste und das zweite Gelenkstück mittels eines Clip-Mechanismus oder/und Druckknopf-Mechanismus und/oder Schraub-Mechanismus voneinander entfernbar oder miteinander verbindbar sind.

Weiterhin kann die erfindungsgemäße Orthese dadurch gekennzeichnet sein, dass sich das zweite Gelenkstück am Ende der Schiene befindet.

Weiterhin kann die erfindungsgemäße Orthese dadurch gekennzeichnet sein, dass das erste Schalenteil eine Ringfassung aufweist, z.B. zur Aufnahme eines Fußes.

Weiterhin kann die erfindungsgemäße Orthese dadurch gekennzeichnet sein, dass zum Reduzieren des Gewichts der Orthese Ausnehmungen in die Schalenteile eingebracht sind.

Weiterhin kann die erfindungsgemäße Orthese dadurch gekennzeichnet sein, dass die Schiene und/oder das

Orthesengelenk teilweise oder vollständig aus Metall, Leichtmetall, insbesondere Aluminium, Magnesium oder/und Edelstahl, Kunststoff, einem Kunststoff-Metallverbund oder einem Verbund mehrerer oder aller vorstehend genannten Materialien besteht.

Weiterhin kann die erfindungsgemäße Orthese dadurch gekennzeichnet sein, dass die Schalenteile teilweise oder vollständig aus einem Glasfaserverbund, Kohlefaserverbund, Basaltfaserverbund oder einem Verbund mehrerer oder aller vorstehend genannten Materialien bestehen.

Weiterhin kann die erfindungsgemäße Orthese dadurch gekennzeichnet sein, dass das erste und/oder das zweite Schalenteil zum Fixieren des ersten bzw. zweiten Körperteils mindestens eine Rasten-schnalle, einen Ratschenverschluss oder ein metallisches Klettverschlusssystem aufweisen.

Weiterhin kann die erfindungsgemäße Orthese dadurch gekennzeichnet sein, dass auf die Schalenteile eine Deckschicht mit verschiedenen Farb- und/oder Mustervarianten auftragbar ist.

Weiterhin kann die erfindungsgemäße Orthese dadurch gekennzeichnet sein, dass die Schalenteile mittels Wassertransferdruck beschichtbar sind.

Weiterhin kann die erfindungsgemäße Orthese dadurch gekennzeichnet sein, dass die Orthese als Sprunggelenksorthese ausgebildet ist, wobei das erste Schalenteil zur Aufnahme eines Fußes und das zweite Schalenteil zur Aufnahme eines Unterschenkels vorgesehen ist, und dass das erste Schalenteil eine gegebenenfalls öffenbare Fersenkappe zur Integration der Ferse des Fußes und zur zuverlässigen Verriegelung der Ferse im Fußteil aufweist.

Weiterhin kann die erfindungsgemäße Orthese dadurch gekennzeichnet sein, dass die Fersenkappe mit einem metallischen Klettverschluss, einem Rastenklettverschluss, einem Schwalbenschwanzverschluss, einem Ratschenverschluss, einem Kunststoffklettbandverschluss oder einem Seilzugsystem verstellbar, insbesondere öffenbar und/oder verschließbar ist.

Weiterhin kann die erfindungsgemäße Orthese dadurch gekennzeichnet sein, dass die Fersenkappe mit einem metallischen Klettverschluss verstellbar, insbesondere öffenbar und/oder verschließbar ist, wobei der metallische Klettverschluss metallische Federstahlbleche mit ausgestanzten Haken und Federstahlbleche mit eingeprägten Ösen aufweist, wobei beim ineinander Greifen die Haken und Ösen aneinander haften.

Weiterhin kann die erfindungsgemäße Orthese dadurch gekennzeichnet sein, dass das Orthesengelenk seitlich um maximal 12° kippbar ist.

Weiterhin kann die erfindungsgemäße Orthese dadurch gekennzeichnet sein, dass die Anschlagscheiben derart einstellbar sind, dass das Sprunggelenk um 30° zu einer horizontalen Ebene nach unten abwinkelbar und um 40° zur horizontalen Ebene nach oben anwinkelbar ist.

Im Folgenden werden einige Beispiele einer erfindungsgemäßen Orthese beschrieben. Die gezeigten einzelnen Merkmale eines Beispiels können dabei auch unabhängig vom konkreten Beispiel und in beliebiger Kombination die vorliegende Erfindung weiterbilden. In der nachfolgenden Beschreibung der Figuren bezeichnen gleiche oder ähnliche Bezugszeichen gleiche oder ähnliche Elemente. Es zeigen
- Figur 1: eine erfindungsgemäße monolaterale Orthese für einen Unterschenkel in verschiedenen Ansichten, a) für einen linken Unterschenkel in einer Rückansicht, b) für einen linken Unterschenkel in einer linken Seitenansicht, c) für einen linken Unterschenkel in einer Perspektivansicht, d) für einen linken Unterschenkel in einer Draufsicht, e) für einen rechten Unterschenkel in einer Rückansicht,
- Figur 2: eine erfindungsgemäße Orthese mit zwei bilateral angeordneten Orthesengelenken für einen Unterschenkel in a) einer linken Seitenansicht, b) einer Rückansicht, c) einer Perspektivansicht,
- Figur 3: eine Einlaminiervariante eines Kugelgelenklagers eines Orthesengelenks in a) einer perspektivischen Explosionsdarstellung, b) einer Seitenansicht, c) einer Vorderansicht,
- Figur 4: eine weitere Variante einer Schiene einer erfindungsgemäßen Orthese in einer perspektivischen Explosionsdarstellung,
- Figur 5: eine Schiene einer erfindungsgemäßen bilateralen Orthese in a) einer Perspektivansicht, b) einer Vorderansicht, c) einer Seitenansicht d) einer Detailansicht,
- Figur 6: eine weitere Variante einer Schiene und eines Orthesengelenks einer erfindungsgemäßen monolateralen Orthese in a) einer perspektivischen Explosionsdarstellung, b) einer Vorderansicht, c) einer Seitenansicht, d) einer Draufsicht, e) einer Detailansicht,
- Figur 7: eine weitere Variante einer Schiene und eines Orthesengelenks einer erfindungsgemäßen monolateralen Orthese in a) einer perspektivischen Explosionsdarstellung, b) einer Seitenansicht, c) einer Vorderansicht, d) einer Draufsicht,
- Figur 8: zeigt eine weitere Variante einer Schiene und eines Orthesengelenks einer erfindungsgemäßen monolateralen Orthese in a) einer perspektivischen Explosionsdarstellung, b) einer Seitenansicht, c) einer Vorderansicht, d) einer Draufsicht,
- Figur 9: einen Ratschenverschluss als Einlaminiervariante in a) einer Einzelansicht der Komponenten, b) einer Seitenansicht in geschlossenem Zustand, c) einer Perspektivansicht in geschlossenem Zustand, d) einer Draufsicht in geschlossenem Zustand,
- Figur 10: die Komponenten eines metallischen Klettverschlusssystems in a) einer Vorderansicht, b) und c) einer Schnittansicht, d) einer Perspektivansicht,
- Figur 11: das metallische Klettverschlusssystem aus Figur 10 in geschlossenem Zustand in a) einer Vorderansicht, b) und c) einer Schnittansicht, d) einer Perspektivansicht,
- Figur 12: ein erstes Schalenteil bzw. eine Ringfassung für einen linken Fuß mit einer Fersenkappe mit Ratschenverschluss in a) einer linken Seitenansicht, b) einer Draufsicht, c) einer Detailansicht.

### Liste der Bezugszeichen:

- 1: erstes Schalenteil/Ringfassung
- 2: zweites Schalenteil
- 3: Orthesengelenk
- 4: erstes Gelenkstück
- 5: Drehpunkt des Orthesengelenks
- 6: Schiene
- 7: zweites Gelenkstück
- 8: zweiter Teil der Schiene 6
- 9: Befestigungspunkte
- 10: Befestigungsfläche
- 11: Kugelschalenteil
- 12: Lagergehäuse
- 13: Einlaminiergehäuse
- 14: Lagergehäuse
- 15: Innenring/Kugelschalenteil
- 16: erste Anschlagscheibe
- 16A: erster Anschlagkeil
- 17: Gelenkachse
- 18: Abdeckscheibe
- 19: zweite Anschlagscheibe
- 19A: zweiter Anschlagkeil
- 20: Kugelgelenklager
- 21: Schrauben
- 22: Gelenkgehäuse
- 23: Einlegekeile
- 24: zweite Anschlagscheibe
- 25: Gewindestifte
- 26: erste Anschlagscheibe
- 27: Gewindeöffnungnen
- 28: Schrauben
- 29: Winkelskala
- 30: Schrauben
- 31: Schienen
- 32: Überstand
- 33: Steg
- 34: Steg
- 35: erste Komponente des Ratschenverschlusses
- 36: zweite Komponente des Ratschenverschlusses
- 37: Überstand
- 38: erste Komponente des metallischen Klettverschlusssystems
- 39: Führungen
- 40: Fläche
- 41: zweite Komponente des metallischen Klettverschlusssystems
- 42: Häkchen
- 43: Randbereiche
- 44: Steg
- 45: schmaler Steg
- 46: schmaler Steg
- 47: schmaler Steg
- 48: Seitenteile
- 49: Gewindestifte
- 51: Bodenelement
- 50: Einlegekeile
- 52: Öffnungen
- 53: Ösen
- 54: Fläche
- 55: Befestigungsaufnahmen
- 56: Aufnahmen
- 60: Fersenkappe
- 61: erste Häkchen
- 62: zweite Häkchen
- 63: Aufnahme für erstes Gelenkstück 4

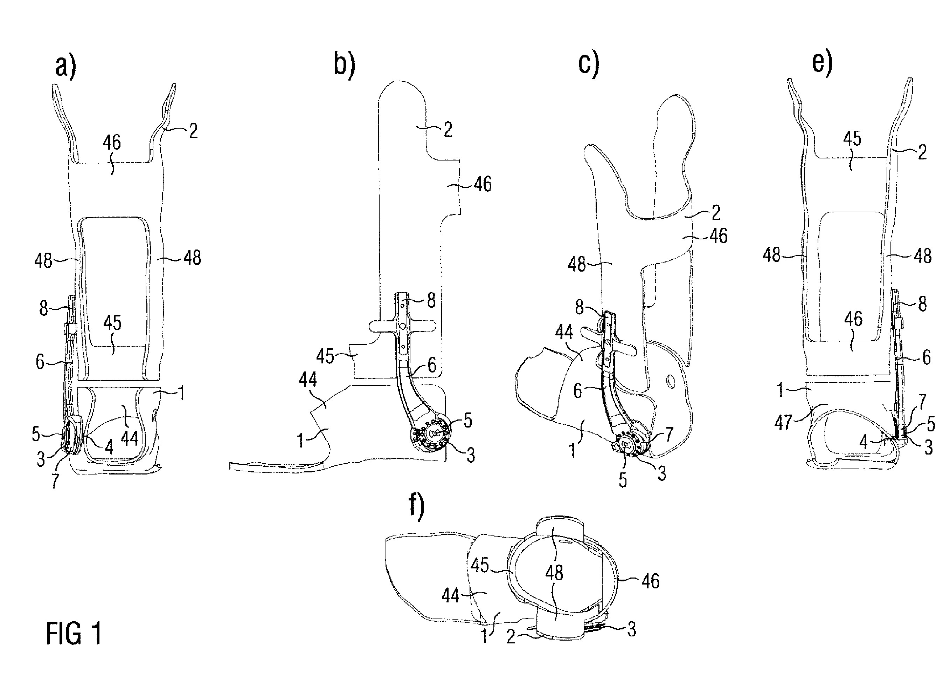

Figur 1 zeigt eine erfindungsgemäße monolaterale Orthese mit einem Orthesengelenk für einen linken bzw. einen rechten Unterschenkel in verschiedenen Ansichten.

Figur 1 a) zeigt eine erfindungsgemäße Orthese für einen linken Unterschenkel mit einem ersten Schalenteil 1 und einem zweiten Schalenteil 2, welche durch ein Orthesengelenk 3 miteinander verbunden sind. Das erste Schalenteil 1 ist als Ringfassung ausgebildet, welche lediglich um den Fuß herum, also im Mittelfußbereich, durch einen Steg 44 geschlossen ist. Im Fersen- und Zehenbereich ist das erste Schalenteil 1 offen. Das zweite Schalenteil 2 besteht im Wesentlichen aus zwei Seitenteilen 48, welche sich von der Höhe des gedachten Sprunggelenks bis etwa zur Höhe des gedachten Knies erstrecken und durch einen schmalen Steg 45 oberhalb des gedachten Sprunggelenks im vorderen Bereich sowie im hinteren Bereich über einen schmalen Steg 46 im oberen Bereich der gedachten Wade miteinander verbunden sind. Das Orthesengelenk 3 besteht aus einem ersten Gelenkstück 4 und einem zweiten Gelenkstück 7. Das Orthesengelenk 3 ist einerseits mittels des ersten Gelenkstücks 4 an der linken Außenseite, also der der Orthese abgewandten Seite, des Schalenteils 1 im Bereich der Ferse derart befestigt, dass der Drehpunkt 5 des Orthesengelenks 3 senkrecht beabstandet zu einer Befestigungsfläche liegt. Diese Befestigungsfläche ist Teil der linken Außenfläche des ersten Schalenteils 1 und wird durch Befestigungspunkte des ersten Gelenkstücks 4 begrenzt. Das zweite Gelenkstück 7 entspricht einem ersten Teil der Schiene 6 und befindet sich in unmittelbarer Nähe des Drehpunkts 5 des Orthesengelenks 3. Die Schiene 6 ist mit ihrem zweiten Teil 8 an der linken Außenseite des zweiten Schalenteils 2 auf Höhe des gedachten Wadenansatzes befestigt.

Figur 1 b) zeigt eine erfindungsgemäße Orthese in einer linken Seitenansicht für einen linken Unterschenkel. Im Vordergrund ist die Schiene 6 der erfindungsgemäßen Orthese zu sehen. An dem ersten Ende der Schiene 6 befindet sich das Orthesengelenk 3 im Fersenbereich des ersten Schalenteils 1, und der zweite Teil 8 der Schiene 6 ist mittig auf dem linken Seitenteil 48 des zweiten Schalenteils 2 befestigt. Der zweite Teil 8 der Schiene 6 ist zur Stabilisierung der Schiene 6 kreuzförmig ausgebildet.

Figur 1 c) zeigt eine erfindungsgemäße Orthese mit einem Orthesengelenk 3 für einen linken Unterschenkel in einer Perspektivansicht mit der Schiene 6 im Vordergrund. Wie in den Figuren 1 a) und 1b) ist hier das Schalenteil 1 im Fersenbereich komplett geöffnet und lediglich als Ringfassung mit einem Steg 44 im Mittelfußbereich ausgebildet. Die Seitenteile 48 des zweiten Schalenteils 2 sind im vorderen Bereich durch einen schmalen Steg 45 direkt oberhalb des gedachten Sprunggelenks und durch einen schmalen Steg 46 auf der Rückseite am oberen Ende des gedachten Wadenbereichs miteinander verbunden.

Figur 1 d) zeigt eine erfindungsgemäße Orthese für einen linken Unterschenkel mit einem Orthesengelenk 3, welches auf der linken Seite der Orthese angeordnet ist, in einer Draufsicht.

Figur 1 e) zeigt eine erfindungsgemäße Orthese mit einem Orthesengelenk 3 auf der rechten Außenseite der Orthese für einen rechten Unterschenkel in einer Rückansicht. Im Gegensatz zu den Beispielen in Figur 1 a), b) und c) ist das Schalenteil 1 im Fersenbereich hier nicht vollständig offen. Stattdessen sind die Seitenbereiche des ersten Schalenteils 1 direkt oberhalb der gedachten Ferse durch einen schmalen Steg 47 miteinander verbunden. Ferner sind die Seitenteile 48 des zweiten Schalenteils 2 auf der Vorderseite durch einen schmalen Steg 45 miteinander verbunden, der sich im Gegensatz zu den in Figur 1 a), b) und c) gezeigten Beispielen direkt unterhalb des gedachten Knies befindet, sowie durch einen schmalen Steg 46 auf der Rückseite und am unteren Ende des Schalenteils 2.

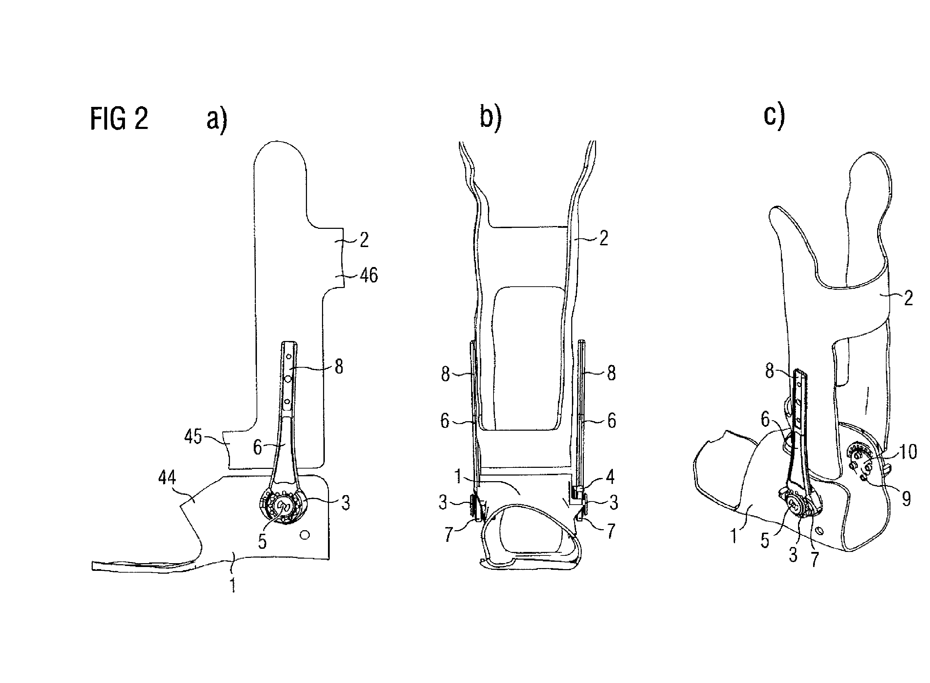

Figur 2 zeigt eine erfindungsgemäße bilaterale Orthese mit zwei bilateral angeordneten Orthesengelenken 3 in verschiedenen Ansichten.

Figur 2 a) zeigt eine erfindungsgemäße Orthese mit zwei bilateral, also beidseitig der Orthese bzw. beidseitig des von der Orthese aufgenommenen, gedachten Körperteils, angeordneten Orthesengelenken 3 für einen linken Unterschenkel in einer linken Seitenansicht. Wie in Figur 1 b) weist die erfindungsgemäße Orthese ein erstes und ein zweites Schalenteil 1 und 2 auf, welche hier aber jeweils durch ein Orthesengelenk 3 auf der linken und ein Orthesengelenk 3 auf der rechten Seite der Orthese miteinander verbunden sind. Die Schalenteile 1 und 2 sowie die Orthesengelenke 3 sind ähnlich wie in Figur 1 b) ausgebildet. Die Drehpunkte 5 der Orthesengelenke 3 befinden sich anders als in Figur 1 b) in unmittelbarer Nähe des gedachten Fußknöchels. Die Orthesengelenke 3 bestehen ebenfalls aus ersten Gelenkstücken 4 und zweiten Gelenkstücken 7, wobei die ersten Gelenkstücke 4 an jeweils der linken und rechten Außenseite des ersten Schalenteils 1 derart befestigt sind, dass die Drehpunkte 5 der Orthesengelenke 3 senkrecht beabstandet zu den Befestigungsflächen liegen. Die Befestigungsflächen werden durch die Befestigungspunkte der Gelenkstücke 4 begrenzt und sind Teil der Außenfläche des ersten Schalenteils 1, an welcher die Gelenkstücke 4 befestigt sind. Die ersten Teile der Schienen 6 bilden die zweiten Gelenkstücke 7, welche sich in unmittelbarer Nähe der Drehpunkte 5 befinden. Die zweiten Teile 8 der Schienen 6 sind jeweils mittig an den Seitenteilen 48 des zweiten Schalenteils 2 auf Höhe des gedachten unteren Wadenbereichs befestigt. Die zweiten Teile 8 der Schienen 6 sind hier lediglich gerade ausgebildet, da im Gegensatz zur monolateralen Orthese aus Figur 1 aufgrund des Vorhandenseins einer zweiten Schiene 6 eine zusätzliche Stabilisierung der einzelnen Schienen 6 durch kreuzförmige zweite Teile 8 nicht notwendig ist.

Figur 2 b) zeigt eine erfindungsgemäße Orthese mit zwei bilateral angeordneten Orthesengelenken 3 in einer Rückansicht für einen rechten Unterschenkel. Die hier gezeigte Orthese ist ähnlich wie in Figur 1 e) aufgebaut und unterscheidet sich lediglich wie schon in Figur 2 a) gezeigt durch eine zweite Schiene 6 und ein zweites Orthesengelenk 3 auf der linken Seite der Orthese sowie durch die Form der Schiene 6.

In Figur 2 c) ist wieder eine erfindungsgemäße Orthese mit zwei bilateral angeordneten Orthesengelenken für einen linken Unterschenkel in einer Perspektivansicht zu sehen. Auf der rechten Innenseite der Orthese, also der dem gedachten Körperteil zugewandte Seite der Orthese, im Bereich des gedachten Fußknöchels sind die Befestigungspunkte 9 für das erste Gelenkstück 4 sowie die Befestigungsfläche 10, welche durch die Befestigungspunkte 9 begrenzt wird, zu sehen.

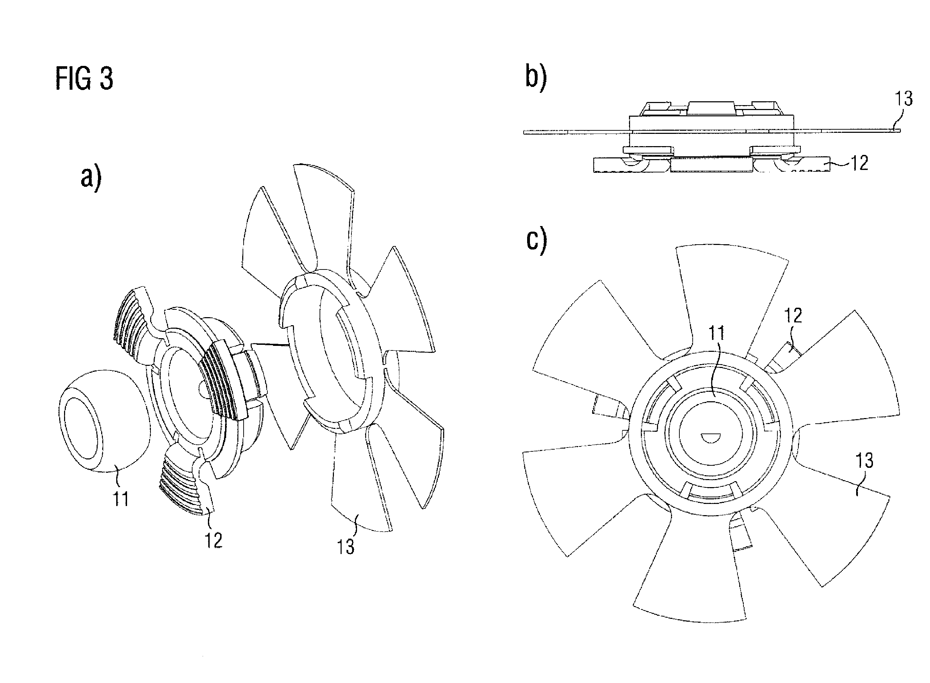

Figur 3 zeigt eine Einlaminiervariante für ein Kugelgelenklager in verschiedenen Ansichten.

Figur 3 a) zeigt eine vorderseitige, perspektivische Explosionsdarstellung eines Kugelgelenklagers mit Einlaminiergehäuse. Im Vordergrund ist ein ringförmiges Kugelschalenteil 11 zu sehen, welches in einem ebenfalls ringförmigen Lagergehäuse 12 mit drei radial angeordneten Flügeln gelagert ist. Das Lagergehäuse 12 wird in ein Einlaminiergehäuse 13 eingeclipst, welches ebenfalls ringförmig mit sechs radial angeordneten Flügeln ausgebildet ist. Üblicherweise wird das Einlaminiergehäuse 13 in das erste Schalenteil 1 einer Orthese einlaminiert. Dabei werden die radial angeordneten Flügel des Einlaminiergehäuses 13 zwischen zwei Materialschichten des ersten Schalenteils 1 gelegt, sodass diese Flügel vollständig vom Material des Schalenteils 1 überdeckt sind und das Einlaminiergehäuse 13 formschlüssig im ersten Schalenteil 1 verankert ist. Anschließend wird das Lagergehäuse 12, in welchem das Kugelschalenteil 11 gelagert ist, in das Einlaminiergehäuse 13 eingeclipst. Des Weiteren ist das Kugelschalenteil 11 mit der Schiene 6 der Orthese fest verbunden und ragt zwischen zwei der drei radial angeordneten Flügel des Lagergehäuses 12 nach oben heraus. Die Schiene 6 ist somit zwischen zwei solcher Flügel des Lagergehäuses 12 beweglich. Die Flügel des Lagergehäuses 12 dienen also als Anschläge für die Bewegung des Orthesengelenks parallel zur Orthese.

Figur 3 b) zeigt eine Seitenansicht einer solchen Einlaminiervariante eines Kugelgelenklagers. Das Lagergehäuse 12, welches das Kugelschalenteil 11 beherbergt, ist vollständig in das Einlaminiergehäuse 13 eingeclipst.

Figur 3 c) zeigt eine Vorderansicht einer Steckverbindung wie in Figur 3 b) des Einlaminiergehäuses 13, in welches das Lagergehäuse 12 eingeclipst ist und das Kugelschalenteil 11 beherbergt.

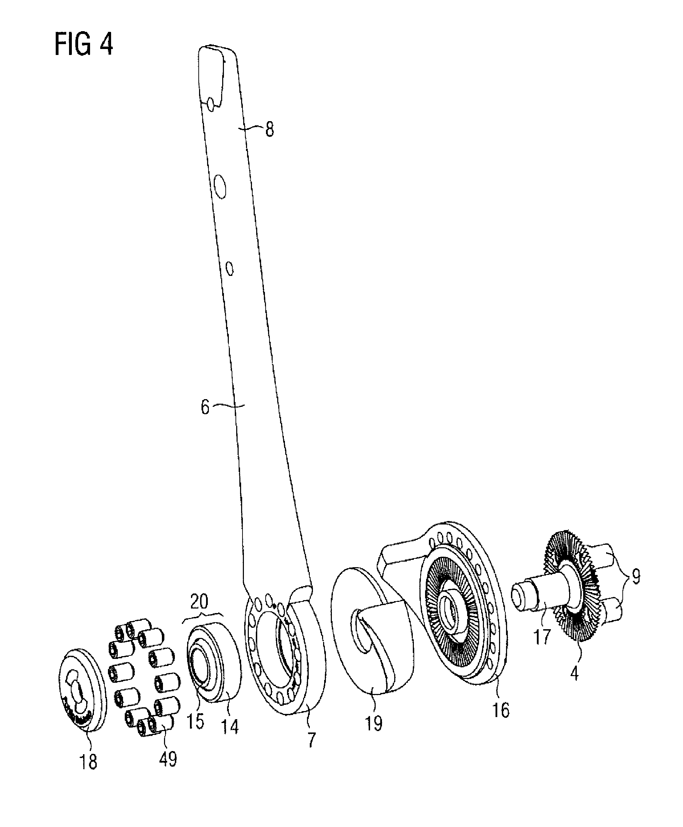

Figur 4 zeigt eine Schiene 6 einer erfindungsgemäßen Orthese mit einem Orthesengelenk 3 in einer perspektivischen Explosionsdarstellung. Auf der dem ersten Schalenteil 1 zugewandten Seite des Orthesengelenks 3 befindet sich das erste Gelenkstück 4, welches auf einer Befestigungsfläche des ersten Schalenteils 1 an den orthesenseitigen Befestigungspunkten 9 befestigt wird. Die Befestigungsfläche 10 (siehe Figur 2 c)) wird dabei durch die Befestigungspunkte 9 des Gelenkstücks 4 begrenzt. Das erste Gelenkstück 4 ist kreisscheibenförmig ausgebildet und liegt parallel zur Befestigungsfläche 10. Es weist ferner senkrecht von der Befestigungsfläche 10 weg weisend eine Gelenkachse 17 auf. Auf der der Orthese abgewandten Seite des Gelenkstücks 4 weist das Gelenkstück 4 um die Achse 17 herum eine radiale Rasterung auf. Auf die Gelenkachse 17 befindet sich weiter eine erste Anschlagscheibe 16 mit einem ersten Anschlagkeil 16A. Die Anschlagscheibe 16 weist sowohl auf der der Orthese abgewandten als auch auf der Orthese zugewandten Seite radiale Rasterungen auf, so dass die Anschlagscheibe 16 mit dem ersten Gelenkstück 4 in einer Ebene paral-1e1 zur Orthese verrastet werden kann, wenn die Anschlagscheibe 16 und das erste Gelenkstück aneinander gedrückt werden. Hinter der Anschlagscheibe 16 auf der der Orthese abgewandten Seite der Anschlagscheibe 16 befindet sich eine zweite Anschlagscheibe 19 mit einem zweiten Anschlagkeil 19A. Auf der zur ersten Anschlagscheibe 16 zugewandten Seite der Anschlagscheibe 19 weist die Anschlagscheibe 19 ebenfalls eine radiale Rasterung auf, sodass die zweite Anschlagscheibe 19 mit der ersten Anschlagscheibe 16 in einer Ebene parallel zur Orthese verrastet werden kann, wenn beide Anschlagscheiben 16 und 19 gegeneinander gedrückt werden. Die Anschlagkeile 16A und 19A begrenzen nun die Bewegung des Orthesengelenks 3 in jeweils beide Richtungen um die Gelenkachse 17, also parallel zur Orthese. Auf der der Orthese abgewandten Seite der Anschlagscheibe 19 befindet sich das zweite Gelenkstück 7, welches auf einem Kugelgelenklager 20 lagert, welches aus einem Innenring 15 mit einer sphärisch, konvex gekrümmten Oberfläche, im weiteren auch Kugelschalenteil genannt, sowie einem Lagergehäuse 14 besteht. Das Lagergehäuse 14 ist dabei fest mit dem zweiten Gelenkstück 7 verbunden, sodass sich das Gelenkstück 7 um das Kugelschalenteil 15 drehen kann. Weiterhin sind in das zweite Gelenkstück 7 Gewindestifte 49 konzentrisch um das Kugelgelenklager 20 eingeschraubt und dienen zur Einstellung des Anschlags für die Bewegung des Orthesengelenks 3 in Richtung der Gelenkachse 17, also senkrecht zur Orthese. Auf der der Orthese abgewandten Seite des zweiten Gelenkstücks 7 ist eine Abdeckscheibe 18 aufgesetzt zur Abdeckung des Orthesengelenks 3.

Mittels der Rasterungen auf den Anschlagscheiben 16 und 19 sowie auf dem ersten Gelenkstück 4 lässt sich die Position der Anschlagkeile 16A und 19A entlang einer Kreisbahn um die Gelenkachse 17 zueinander und bezüglich des ersten Gelenkstücks 4 in 5°-Schritten einstellen. Damit lassen sich Darsalextension, also der Bewegungsbereich für die Bewegung des Fußes in Richtung des Spanns, und Plantarflexion, also der Bewegungsbereich für die Bewegung des Fußes in Richtung der Fußsohle, in 5°-Schritten individuell einstellen.

Der Bewegungsbereich des Orthesengelenks in senkrechter Richtung zu den Schalenteilen der Orthese lässt sich mittels der Gewindestifte 49 individuell einstellen. Dabei können die Gewindestifte 49 unterschiedlich weit in das zweite Gelenkstück 7 eingeschraubt werden, wodurch die Stellung der Schiene 6 auf dem Kugelschalenteil 15 in einen bestimmten Winkelbereich gezwungen wird.

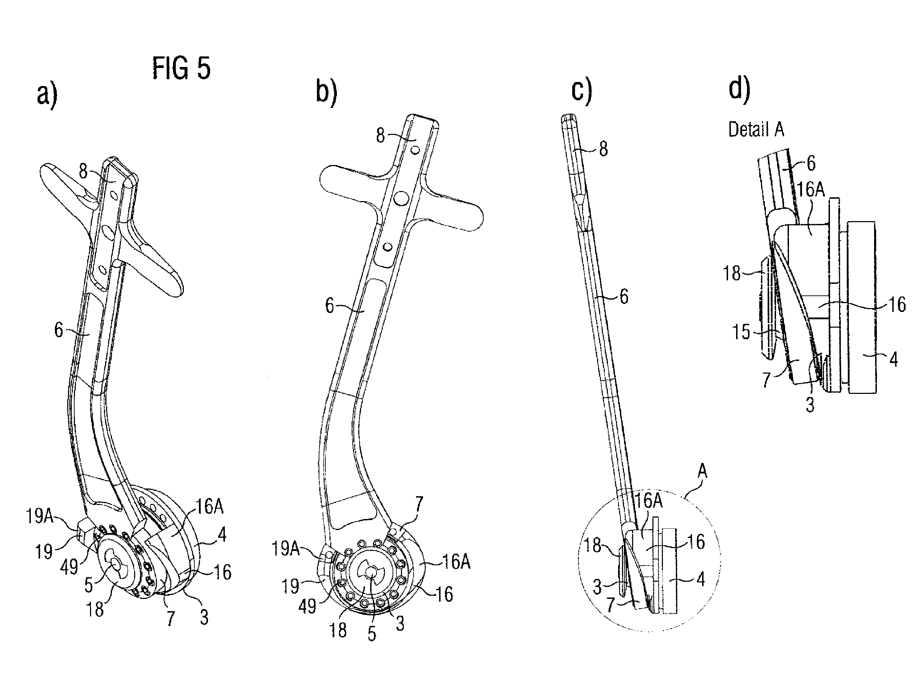

Figur 5 zeigt eine Schiene 6 mit einem Orthesengelenk 3 einer erfindungsgemäßen monolateralen Orthese in verschiedenen Ansichten. Das Orthesengelenk 3 ist hier genau wie in Figur 4 aufgebaut. Lediglich die Form der Schienen 6 in Figur 5 unterscheidet sich von der in Figur 4.

Figur 5 a) zeigt eine längliche Schiene 6 in einer vorderseitigen Perspektivansicht mit einem zweiten Teil 8, welcher an ein Seitenteil 48 eines zweiten Schalenteils 2 einer erfindungsgemäßen Orthese befestigbar ist, und mit einem dem zweiten Teil gegenüberliegenden ersten Teil, welcher als zweites Gelenkstück 3 einen Teil des Orthesengelenks 3 bildet. Der zweite Teil 8 der Schiene 6 ist kreuzförmig ausgebildet sowie um einen Winkel zwischen 0° und 90° nach hinten weggebogen. Das Orthesengelenk 3 in Figur 5 a) weist weiter wie in Figur 4 ein erstes Gelenkstück 4, welches an der Außenseite des ersten Schalenteils 1 befestigbar ist, zwischen dem ersten Gelenkstück 4 und dem zweiten Gelenkstück 7 eine erste Anschlagscheibe 16 mit einem ersten Anschlagkeil 16A und eine zweite Anschlagscheibe 19 mit einem zweiten Anschlagkeil 19A, sowie auf der der Orthese abgewandten Seite des zweiten Gelenkstücks 7 eine Abdeckscheibe 18 auf.

Figur 5 b) zeigt die Schiene 6 mit Orthesengelenk 3 in einer Vorderansicht. Im Mittelpunkt des Orthesengelenks 3 befindet sich der Drehpunkt 5. Im Vordergrund ist die Abdeckscheibe 18 des Orthesengelenks zu sehen. Dahinter, in einer Richtung, die in die Ebene des Blattes hinein zeigt, befindet sich das zweite Gelenkstück 7, welches auf dem Kugelgelenklager 20 gelagert ist. Der Anschlag des Orthesengelenks für die Bewegung senkrecht zur Orthese ist mittels Gewindestiften 49 einstellbar, welche in konzentrisch um die Gelenkachse im zweiten Gelenkstück 7 angeordnete Gewinde eingeschraubt sind. Hinter dem kugelgelagerten Gelenkstück 7 befindet sich eine zweite und eine erste Anschlagscheibe 19 und 16. Der zweite Teil 8 der Schiene 6 ist kreuzförmig ausgebildet sowie um einen Winkel zwischen 0° und 90° nach hinten weggebogen.

Figur 5 c) zeigt die gleiche Schiene 6 mit einem Orthesengelenk 3 wie die Figuren 5 a) und 5b) in einer Seitenansicht mit dem ersten Anschlagkeil 16A der ersten Anschlagscheibe 16 im Vordergrund. Die Gelenkachse 17 wird auf der der Orthese abgewandten Seite durch die Abdeckscheibe 18 gesichert. Ferner ist zu sehen, dass die auf einem Kugelgelenk mit einem Kugelschalenteil 15 gelagerte Schiene 6 zwischen der Abdeckscheibe 18 und der Anschlagscheibe 19 in einer Richtung senkrecht zur Orthese und zwischen dem ersten Anschlagkeil 16A und dem zweiten Anschlagkeil 19A in einer Richtung parallel zur Orthese beweglich ist.

Figur 5 d) zeigt das Orthesengelenk 3 in der gleichen Ansicht wie in Figur 5 a) in einer Detailansicht.

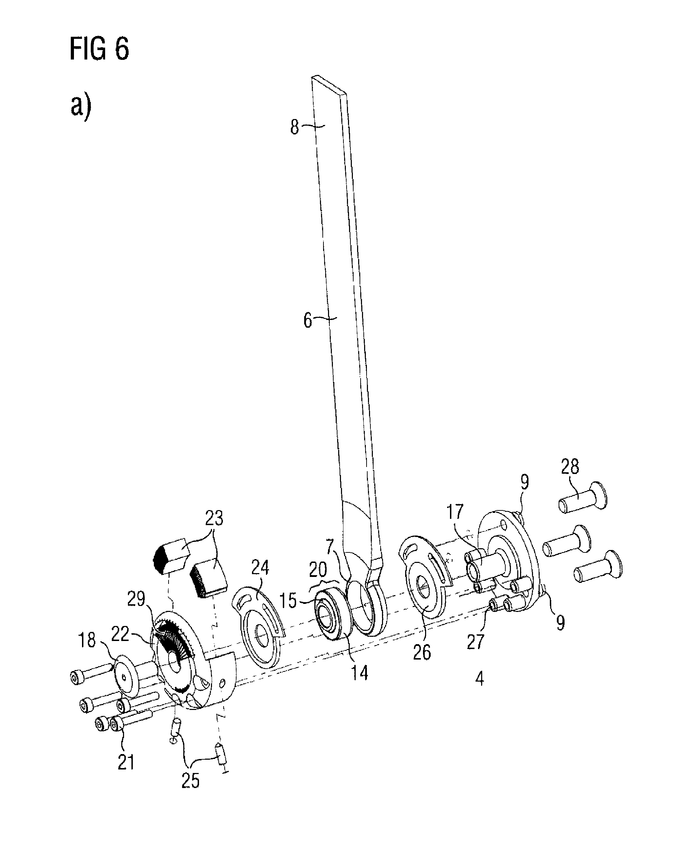

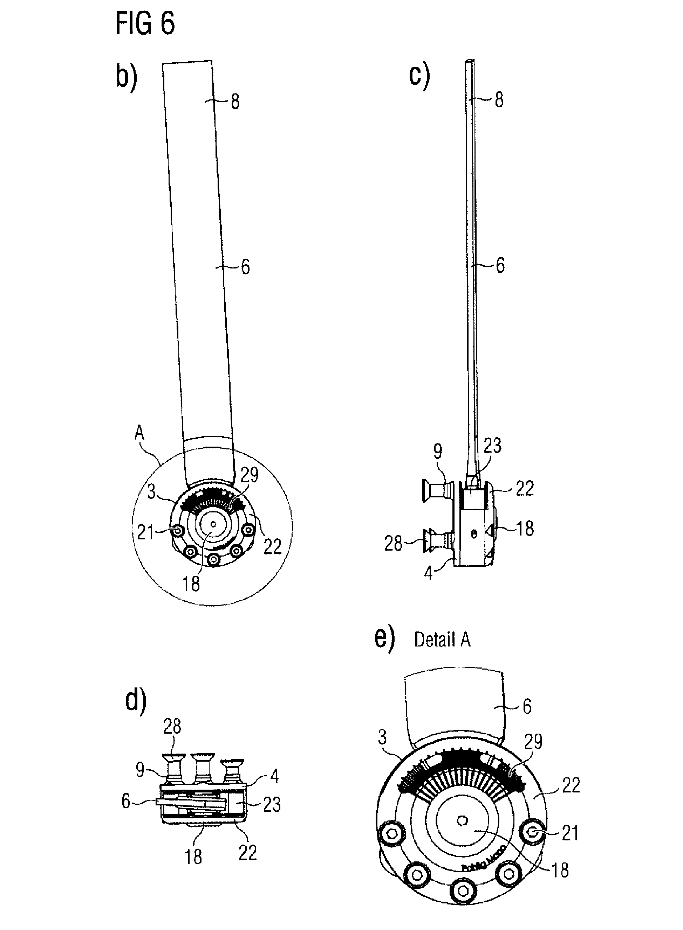

Figur 6 zeigt eine weitere Variante eines Orthesengelenks 3 einer erfindungsgemäßen monolateralen Orthese in verschiedenen Ansichten.

Figur 6 a) zeigt das Orthesengelenk 3 in einer perspektivischen Explosionsdarstellung. Auch bei dieser Variante ist das Gelenkstück 4 kreisscheibenförmig ausgebildet und wird an seinen orthesenseitigen Befestigungspunkten 9 mittels Schrauben 28 an eine Befestigungsfläche 10 des ersten Schalenteils 1 angeschraubt. Das Gelenkstück 4 weist im Mittelpunkt der Kreisscheibe ebenfalls eine senkrecht von der Befestigungsfläche 10 und der Orthese weg weisende Gelenkachse 17 auf. In der unteren Hälfte des Gelenkstücks 4 sind senkrecht zur Befestigungsfläche 10 und parallel zur Gelenkachse 7 auf der der Orthese abgewandten Seite des Gelenkstücks 4 zylindrische Gewindeöffnungen 27 konzentrisch um die Gelenkachse 17 angeordnet. In diese Gewindeöffnungen 27 werden Schrauben 21 zur Befestigung des Gelenkgehäuses 22 eingeschraubt. Auf der Gelenkachse 17 befindet sich weiter eine erste Anschlagscheibe 26. Auf der der Orthese abgewandten Seite der ersten Anschlagscheibe 26 befindet sich das zweite Gelenkstück 7, welches das Kugelgelenklager 20 umfasst. Das Kugelgelenklager 20 besteht auch hier aus einem Kugelschalenteil 15, welche sich in einem Lagergehäuse 14 befindet. Das Lagergehäuse 14 ist dabei fest mit dem zweiten gelenkstück 7 verbunden, sodass sich das Gelenkstück 7 um das Kugelschalenteil 15 drehen kann. Auf der der Orthese abgewandten Seite des Kugelgelenklagers 20 befindet sich eine zweite Anschlagscheibe 24. Auf der der Orthese abgewandten Seite der zweiten Anschlagscheibe 24 befindet sich auf der Gelenkachse 17 das Gelenkgehäuse 22, welches mittels Schrauben 21, welche in die Gewindeöffnungen 27 greifen, befestigt wird, Die Schrauben 21 werden dabei nicht durch die Anschlagscheiben 24 und 26 und das zweite Gelenkstück 7 geführt. Die Anschlagscheiben 24 und 26 weisen dazu im Bereich der Schrauben 21 Aussparungen auf. An der der Orthese abgewandten Seite des Gelenkgehäuses 22 wird die Gelenkachse 17 mit einer Abdeckscheibe 18 als Achssicherung gesichert. Das Gelenkgehäuse 22 weist ferner zwei kreisbogenförmige Einlegekeile 23 auf, welche auf einer Kreisbahn um die und oberhalb der Gelenkachse 17 positioniert sind. Diese Einlegekeile 23 dienen zur Einstellung des Bewegungsbereichs des Orthesengelenks 3, d.h. zur Einstellung der Dorsalextension und der Plantarflexion. Die Einstellung geschieht über Gewindestifte 25, welche jeweils diametral zueinander seitlich und senkrecht in das Gelenkgehäuse 22 sowie jeweils in die Einlegekeile 23 eingeschraubt werden. Durch Hereindrehen oder Herausdrehen dieser Gewindestifte 25 verschiebt sich die Position der Einlegekeile 23 auf einem Kreisbogen im oberen Bereich der und um die Gelenkachse 17, wodurch der mögliche Bewegungsbereich des Orthesengelenks 3 verkleiner oder vergrößert wird. Die Position der Einlegekeile 23 kann auf einer Rasterung an der der Orthese abgewandten Außenseite des Gelenkgehäuses abgelesen werden. Diese Rasterung 29 dient somit als Winkelskala 29 zur Einstellung des Bewegungsbereichs des Orthesengelenks für die Bewegung parallel zur Orthese.

Figur 6 b) zeigt die gleiche Variante wie Figur 6 a) eines Orthesengelenks 3 in einer Vorderansicht mit der Abdeckscheibe 18, der darüberliegenden Winkelskala 29 und der im unteren Bereich des Gelenkgehäuses 22 angeordneten Schrauben zur Befestigung des Gelenkgehäuses 22 im Vordergrund.

Figur 6 c) zeigt die gleiche Variante eines Orthesengelenks 3 wie in den Figuren 6 a) und b) in einer Seitenansicht, wobei im linken Bildbereich die Schrauben 28 zu sehen sind, welche in die Befestigungspunkte 9 eingeschraubt sind und zur Befestigung des Orthesengelenks 3 am ersten Schalenteil 1 dienen.

Figur 6 d) zeigt die gleiche Variante eines Orthesengelenks 3 wie in den Figuren 6 a) bis 6c) in einer Draufsicht.

Figur 6 e) zeigt eine Detailansicht des Orthesengelenks 3 in einer Vorderansicht wie in Figur 6 b).

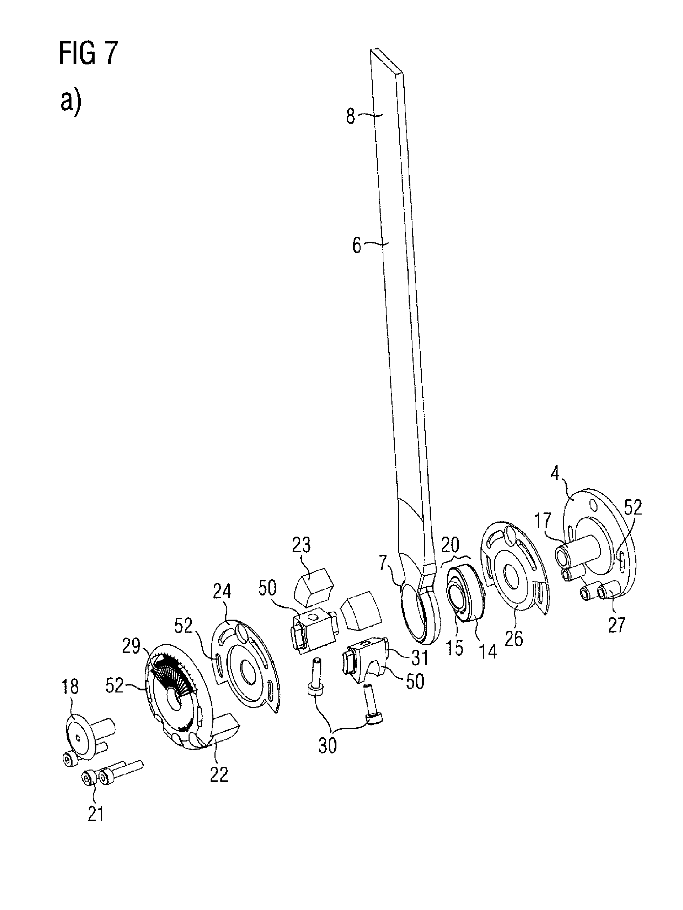

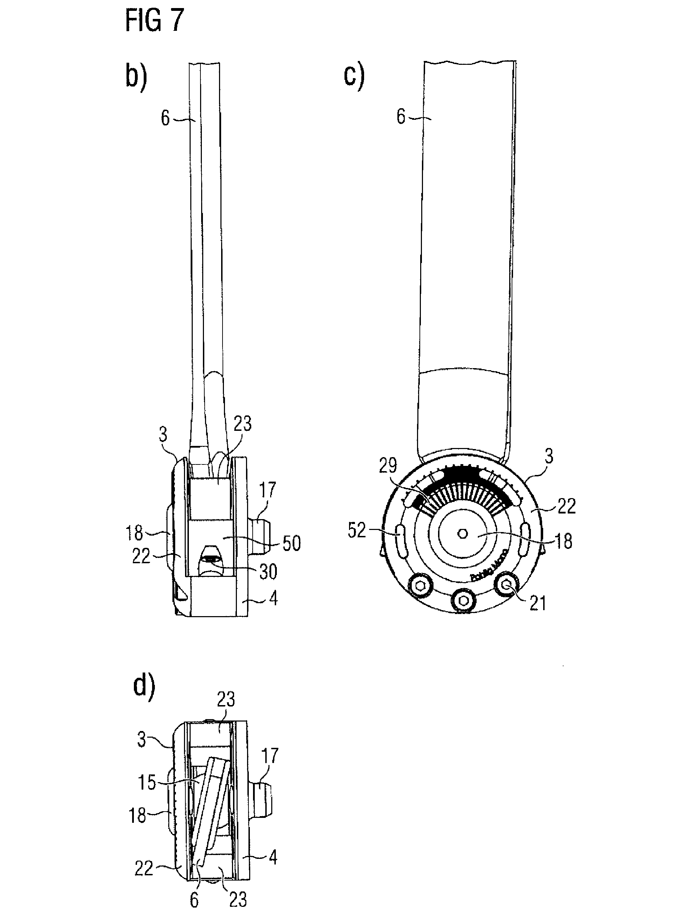

Figur 7 zeigt eine weitere Variante eines Orthesengelenks 3 einer erfindungsgemäßen monolateralen Orthese in verschiedenen Ansichten.

Figur 7 a) zeigt ein Orthesengelenk 3 in einer perspektivischen Explosionsdarstellung. Wie in Figur 6 a) weist das Orthesengelenk 3 in Figur 7 a) ein kreisscheibenförmiges erstes Gelenkstück 4 auf, welches im Mittelpunkt auf seiner der Orthese abgewandten Seite senkrecht eine Gelenkachse 17 sowie in seinem unteren Bereich konzentrisch um die Gelenkachse 17 und parallel zur Gelenkachse 17 angeordnete zylindrische Gewindeöffnungen 27 aufweist. Auf der Gelenkachse 17 befindet sich weiter eine erste Anschlagscheibe 26 sowie das zweite Gelenkstück 7, welches wieder auf dem Kugelgelenklager 20 lagert. Auch hier besteht das Kugelgelenklager 20 aus einem ringförmigen Kugelschalenteil 15, welche sich in einem Lagergehäuse 14 befindet. Das Lagergehäuse 14 ist dabei fest mit dem zweiten Gelenkstück 7 verbunden, sodass sich das Gelenkstück 7 um das Kugelschalenteil 15 drehen kann. Ferner weist das Orthesengelenk 3 der Figur 7 eine zweite Anschlagscheibe 24 und ein Gelenkgehäuse 22 auf, welche sich beide auf der Gelenkachse 17 befinden. Das Gelenkgehäuse 22 wird mit Schrauben 21 befestigt, welche konzentrisch um die und unterhalb der Gelenkachse 17 angeordnet sind und in die zylindrischen Gewindeöffnungen 27 eingeschraubt werden. Die Schrauben 21 führen dabei nicht durch die Anschlagscheiben 24 und 26 und das zweite Gelenkstück 7. Die Anschlagscheiben 24 und 26 weisen im unteren Bereich, also im Bereich der Schrauben 28 eine Aussparung auf. Die Gelenkachse 17 wird zusätzlich mit einer Abdeckscheibe 18 als Achssicherung gesichert. Die Anschlagscheiben 24 und 26 sowie das Gelenkgehäuse 22 und das Gelenkstück 4 weisen diametral zueinander um die Gelenkachse 17 kreisbogenförmige Öffnungen 52 auf, welche zur Halterung von Einlegekeilen 50 dienen. Diese Einlegekeile 50 befinden sich zwischen der zweiten Anschlagscheibe 24 und dem zweiten Gelenkstück 7. Die Einlegekeile 50 selbst sind ebenfalls kreisbogenförmig und werden von parallel zur Gelenkachse ausgerichteten Schienen 31 gehalten, welche einen kreisbogenförmigen Querschnitt aufweisen und durch die kreisbogenförmigen Öffnungen 52 der Anschlagscheiben 24 und 26 sowie des Gelenkgehäuses 22 und des ersten Gelenkstücks 4 geführt werden. Auf diese Einlegekeile 50 werden weitere Einlegekeile 23 geschraubt, deren Position auf einem Kreisbogen um die Gelenkachse 17 mittels der Schrauben 30 verändert werden kann. Dadurch ist die Einstellung der Position und eine Verkleinerung oder Vergrößerung des Bewegungsbereichs möglich. Die Gradeinstellung ist wie im vorherigen Beispiel in Figur 6 auf eine sich auf der der Orthese abgewandten Seite des Gelenkgehäuses 22 befindenden Rasterung, welche als Winkelskala 29 dient, ablesbar. Die Schiene 6 ist zwischen den Einlegekeilen 50 um den Drehpunkt 5 des Orthesengelenks frei beweglich.

Figur 7 b) zeigt die gleiche Variante eines Orthesengelenks 3 wie in Figur 7 a) in einer Seitenansicht mit den Einlegekeilen 50 und 23 sowie den Schrauben 30 zur Einstellung der Position der Einlegekeile 23 auf einem Kreisbogen um die Gelenkachse 17.

Figur 7 c) zeigt die gleiche Variante eines Orthesengelenks 3 wie in den Figuren 7 a) und b) in einer Vorderansicht mit der Abdeckscheibe 18, der Winkelskala 29, den kreisbogenförmigen Öffnungen 52, durch welche die Schienen 31 zur Halterung der Einlegekeile 50 geführt werden, die Schrauben 21 unterhalb der Gelenkachse 17 zur Befestigung des Gelenkgehäuses 22 im Vordergrund.

Figur 7 d) zeigt die gleiche Variante eines Orthesengelenks 3 wie in den Figuren 7 a) bis c) in einer Draufsicht. Im Vordergrund ist der zweite Teil 8 der Schiene 6 zu sehen. Das Gelenkgehäuse 22 ist nach oben hin offen, so dass sich die Schiene 6 innerhalb des Bewegungsbereichs frei parallel und senkrecht zur Orthese bewegen lässt. Ferner ist zu erkennen, dass der Bewegungsbereich der Schiene parallel zur Gelenkachse 17 nur sowohl durch das erste Gelenkstück 4 als auch durch das Gelenkgehäuse 22 begrenzt wird.

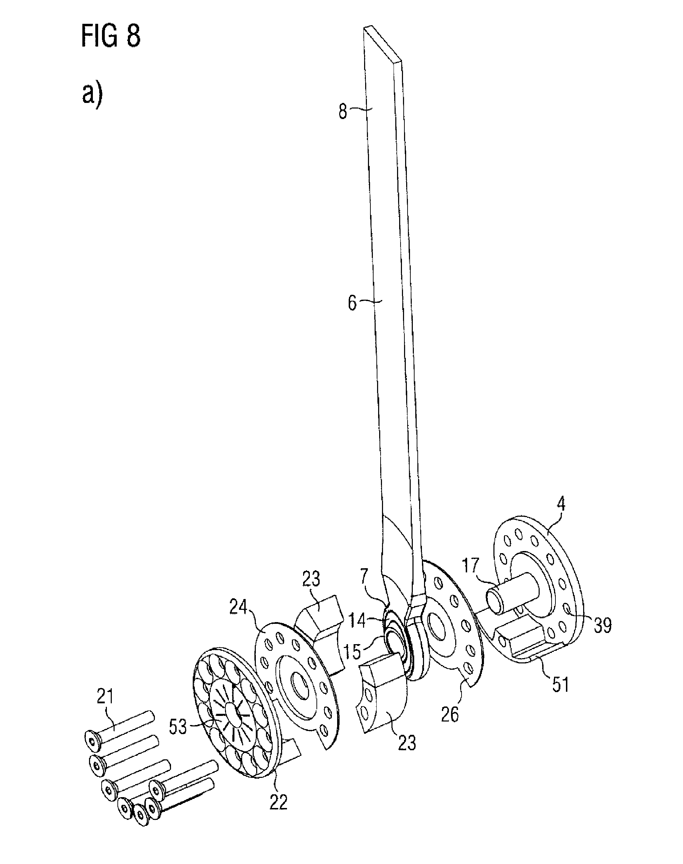

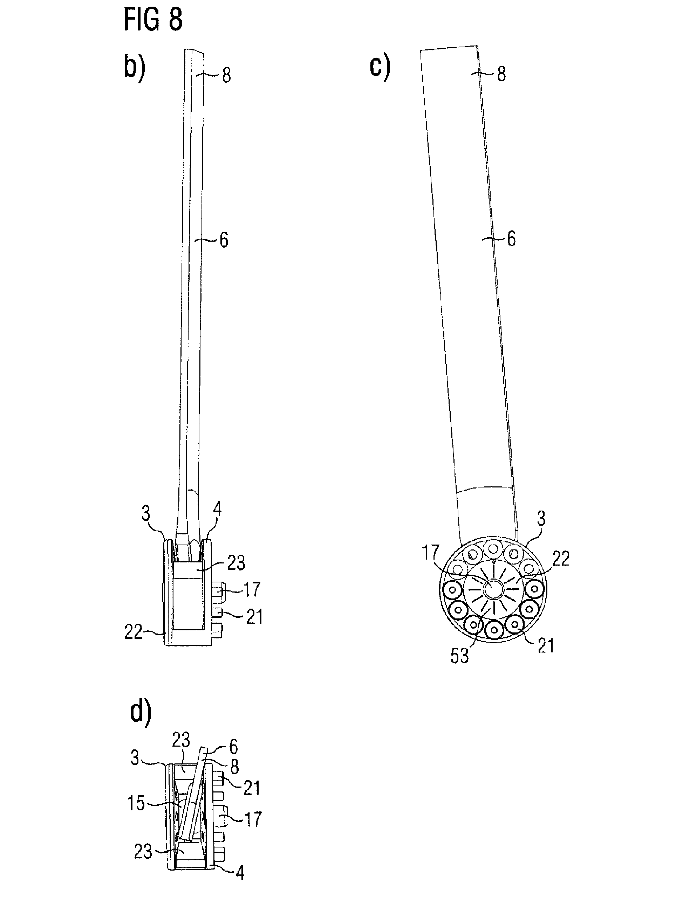

Figur 8 zeigt eine weitere Variante eines Orthesengelenks 3 einer erfindungsgemäßen monolateralen Orthese in verschiedenen Ansichten.

Figur 8 a) zeigt eine perspektivische Explosionsdarstellung dieser weiteren Variante eines Orthesengelenks 3. Wie schon in den vorangegangenen Figuren 4 bis 7 weist das Orthesengelenk 3 ein kreisscheibenförmiges erstes Gelenkstück 4 auf mit einer im Mittelpunkt angeordneten Gelenkachse 17, welche sich auf der der Orthese abgewandten Seite des Gelenkstücks 4 und senkrecht zum Gelenkstück 4 bzw. zur Befestigungsfläche 10 befindet. Um die Gelenkachse 17 herum sind auf dem ersten Gelenkstück 4 Führungen 39 für die Schrauben 21 zur Befestigung des Gelenkgehäuses 22 am Gelenkstück 4 und des Orthesengelenks 3 auf der Befestigungsfläche 10 des ersten Schalenteils 1 konzentrisch angebracht. Ferner befindet sich unterhalb der Gelenkachse 17 auf der der Orthese abgewandten Seite des Gelenkstücks 4 ein Bodenelement 51, welches zusammen mit dem Gelenkgehäuse 22 das Gehäuse des Orthesengelenks 3 bildet. Dieses Bodenelement 51 dient zur Führung der weiteren Gelenksbestandteile auf der Gelenkachse 17. Auf der Gelenkachse 17 befindet sich nun eine erste Anschlagscheibe 26, welche im Bereich des Bodenelements 51 eine Aussparung aufweist. In die Anschlagscheibe 26 sind um die Gelenkachse 17 herum konzentrische Öffnungen eingelassen, durch welche die Schrauben 21 zur Befestigung des Gelenkgehäuses 22 geführt werden. Auf der der Orthese abgewandten Seite der Anschlagscheibe 26 befindet sich weiter das zweite Gelenkstück 7 auf der Gelenkachse 17, wobei das zweite Gelenkstück 7 auf dem Kugelgelenklager 20 lagert. Auch hier besteht das Kugelgelenklager aus einem ringförmigen Kugelschalenteil 15 sowie einem Lagergehäuse 14, in welchem sich das Kugelschalenteil 15 befindet. Das Lagergehäuse 14 ist dabei fest mit dem zweiten Gelenkstück 7 verbunden, sodass sich das Gelenkstück 7 um das Kugelschalenteil 15 drehen kann. Auf der der Orthese abgewandten Seite des zweiten Gelenkstücks 7 befindet sich weiter eine zweite Anschlagscheibe 24 auf der Gelenkachse 17. Im unteren Bereich, also im Bereich des Bodenelements 51 weisen die kreisscheibenförmigen Anschlagscheiben 24 und 26 Aussparungen auf. Auf der der Orthese abgewandten Seite der zweiten Anschlagscheibe 24 befindet sich das Gelenkgehäuse 22 und wird mit den Befestigungsschrauben 21, welche jeweils durch die Anschlagscheiben 24 und 26 hindurchgeführt werden, am ersten Gelenkstück 4 angeschraubt. Das Gelenkgehäuse 22 hat ferner im Bereich des Bodenelements 51, welches am ersten Gelenkstück 4 angeordnet ist, eine Aussparung. Das Gelenkgehäuse 22 ist nach oben hin offen, so dass sich die Schiene 6 innerhalb des Bewegungsbereichs frei parallel und senkrecht zur Orthese bewegen lässt. Zwischen dem zweiten Gelenkstück 7 und der zweiten Anschlagscheibe 24 sind weiter beidseitig der Gelenkachse 17 kreisbogenförmige Einlegekeile 23 eingesetzt. Diese weisen jeweils zwei Schraubenführungen auf, durch welche je nach Position der Einlegekeile 23 entlang eines Kreisbogens um die Gelenkachse 17 jeweils zwei benachbarte Schrauben der Befestigungsschrauben 21 ebenfalls geführt werden können. Anders ausgedrückt, je nachdem welche jeweils zwei benachbarten Schrauben der konzentrisch um die Gelenkachse 17 angeordneten Befestigungsschrauben 21 durch die Einlegekeile 23 geführt werden, verändert sich der zulässige Bewegungsbereich des Orthesengelenks 3 in einer Ebene parallel zur Orthese bzw. zur Befestigungsfläche 10. Hierdurch kann also der Bewegungsbereich der Orthese um ein Vielfaches des Abstandes der Befestigungsschrauben 21 verstellt werden. Der Öffnungswinkel des Bewegungsbereichs lässt sich auf einer Winkelskala 29 ablesen, die konzentrisch um die Gelenkachse 17 und auf der der Orthese abgewandten Seite des Gelenkgehäuses 22 angebracht ist.

Figur 8 b) zeigt eine Seitenansicht des Orthesengelenks 3 aus Figur 8 a) mit den Einlegekeilen 23 im Vordergrund. Im rechten Bildbereich sind die Verlängerung der Gelenkachse 17 zum Schalenteil 1 hin sowie die Enden Schrauben 21 zur Befestigung des Gelenkgehäuses 22 an dem ersten Gelenkstück 4 bzw. zur Befestigung des Orthesengelenks 3 auf der Befestigungsfläche 10 des ersten Schalenteils 1 zu sehen.

Figur 8 c) zeigt eine Vorderansicht des Orthesengelenks 3 aus Figur 8 a) und c) einer erfindungsgemäßen Orthese mit dem Gelenkgehäuse 22, der Gelenkachse 17, der Winkelskala 29, die konzentrisch um die Gelenkachse 17 angebracht ist, und den Schrauben 21 zur Befestigung des Gelenkgehäuses 22 an dem ersten Gelenkstück 4 bzw. zur Befestigung des Orthesengelenks 3 auf der Befestigungsfläche 10 des ersten Schalenteils 1 sowie zur Einstellung der Position der Einlegekeile 23.

Figur 8 d) zeigt das Orthesengelenk aus den Figuren 8 a) bis c) in einer Draufsicht. Im Vordergrund ist der zweite Teil 8 der Schiene 6 zu sehen.

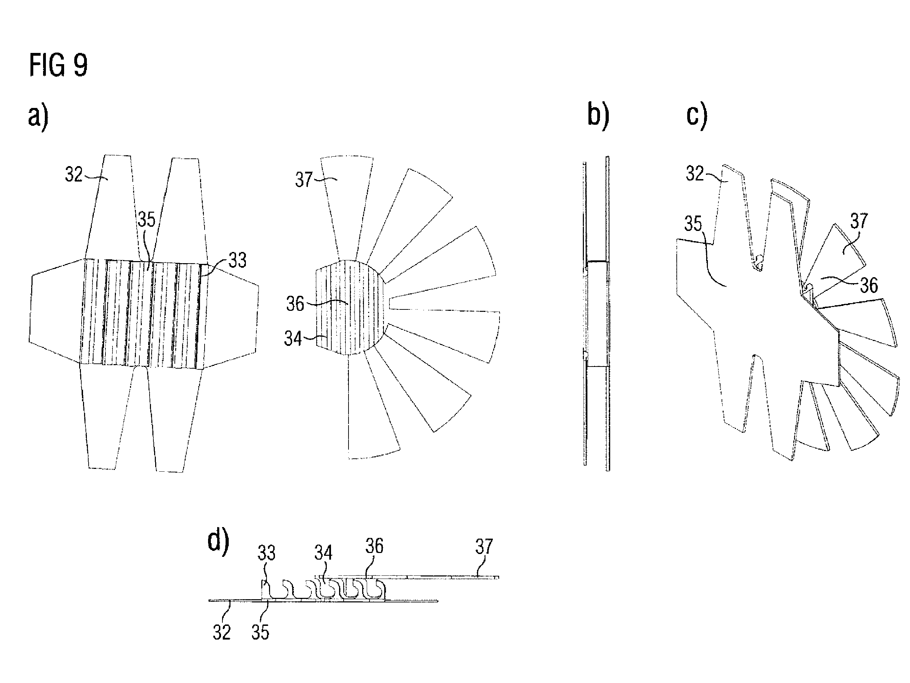

Figur 9 zeigt einen Ratschenverschluss zum Öffnen bzw. Verschließen der Orthese bzw. des zweiten Schalenteils 2 beispielsweise im gedachten Schienbeinbereich oder gegebenenfalls im gedachten Fersenbereich des ersten Schalenteils 1.

Figur 9 a) zeigt eine Ansicht beider Komponenten des Ratschenverschlusses in der Einlaminiervariante. Die zwei Komponenten 35 und 36 des Ratschenverschlusses werden jeweils beispielsweise an eines der Seitenteile 48 angebracht. In dem Fall der Einlaminiervariante sind die Komponenten des Ratschenverschlusses an ihren Überständen 32 und 37 in das Material des Schalenteils eingearbeitet bzw. einlaminiert. Alternativ ist es möglich, die beiden Komponenten jeweils an ihren Überständen an die Schalenteile anzuschrauben bzw. anzunieten. Zum Verschließen des Ratschenverschlusses können beide Komponenten 35 und 36 ineinander verhakt werden. Dazu weisen die Komponenten 35 und 36 auf einer Verschlussfläche jeweils hinterschnittene, parallel laufende Stege 33 bzw. 34 auf, welche beim Übereinanderlegen beider Komponenten miteinander verhaken.

Figur 9 b) zeigt eine Seitenansicht des Ratschenverschlusses im geschlossenen Zustand.

Figur 9 c) zeigt den Ratschenverschluss im geschlossenen Zustand in einer Perspektivansicht mit der Komponente 35 im Vordergrund.

Figur 9 d) zeigt eine Querschnittsansicht des Ratschenverschlusses im geschlossenen Zustand. Im mittleren Bildbereich sind die hakenförmigen Querschnittsflächen der Stege 33 und 34 der Komponenten 35 und 36 des Ratschenverschlusses zu sehen.

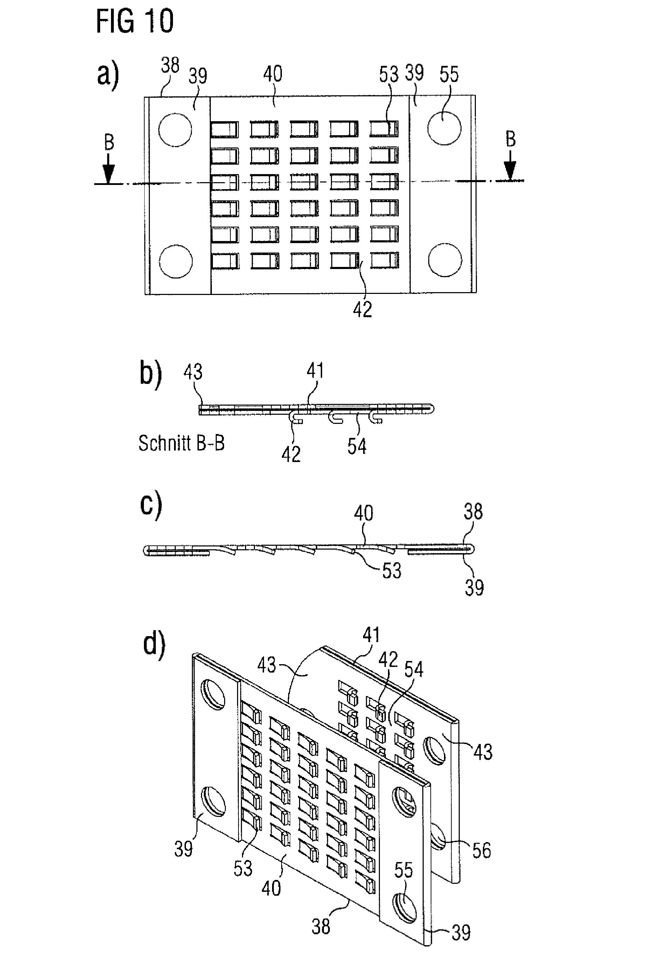

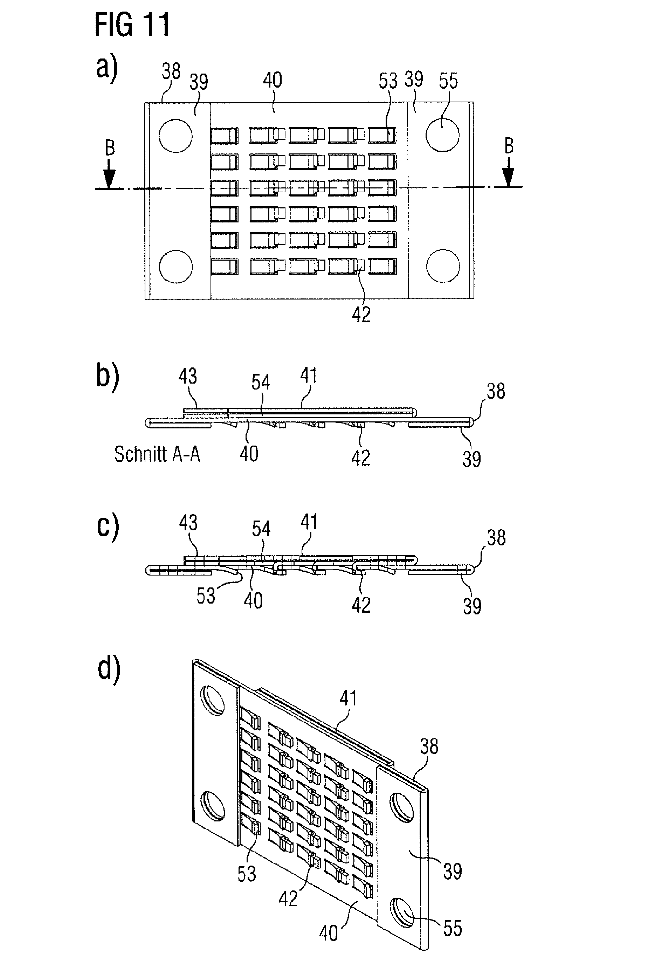

Figur 10 und Figur 11 zeigen ein metallisches Klettverschlusssystem, welches alternativ zu einem Ratschenverschluss zum Öffnen bzw. Verschließen der Schalenteile der Orthese verwendet werden kann.

Figur 10 zeigt die einzelnen Komponenten eines metallischen Klettverschlusssystems im geöffneten Zustand.

Figur 10 a) zeigt die erste Komponente 38 des metallischen Klettverschlusssystems, welche im mittleren Bereich eine Fläche 40 mit rasterartig eingelassenen Ösen 53 aufweist. In den Randbereichen 39, welche sich beidseitig der Fläche 40 befinden, sind jeweils zwei Befestigungsaufnahmen 55 eingelassen.

Figur 10 b) zeigt die zweite Komponente 41 des metallischen Klettverschlusssystems, wobei sich mittig in dieser Komponente 41 eine Fläche 54 mit rasterartig angeordneten Häkchen 42 befindet sowie in den Randbereichen 43 Aufnahmen 56 für Befestigungsmittel.

Figur 10 c) zeigt eine Schnittansicht der ersten Komponente 38 des metallischen Klettverschlusssystems entlang der Schnittlinie B-B, die in Figur 10 a) eingezeichnet ist, mit den Ösen 53, welche rasterartig auf der Fläche 40 angeordnet sind.

Figur 10 d) zeigt beide Komponenten 38 und 41 des metallischen Klettverschlusssystems im geöffneten Zustand. Das Raster der rasterartig angeordneten Ösen 53 auf der Fläche 40 der ersten Komponente 38 des metallischen Klettverschlusssystems stimmt mit dem Raster der rasterartig angeordneten Häkchen 42 auf der Fläche 54 der zweiten Komponente 41 des metallischen Klettverschlusssystems überein.

Figur 11 zeigt das metallische Klettverschlusssystem im geschlossenen Zustand.

Figur 11 a) zeigt beide Komponenten 38 und 41 des metallischen Klettverschlusssystems im geschlossenen Zustand in einer Vorderansicht. Im geschlossenen Zustand greifen die Häkchen 42 der zweiten Komponente 41 durch die Ösen 53, welche rasterartig auf der Fläche 40 der ersten Komponente 38 des metallischen Klettverschlusssystems angeordnet sind.

Figur 11 b) zeigt eine Draufsicht auf das metallische Klettverschlusssystem im geschlossenen Zustand.

Figur 11 c) zeigt einen Schnitt entlang der Schnittlinie A-A, welche in Figur 11 a) eingezeichnet ist, des metallischen Klettverschlusssystems im geschlossenen Zustand.

Figur 11 d) zeigt eine Perspektivansicht des metallischen Klettverschlusssystems im geschlossenen Zustand mit der Komponente 38 im Vordergrund.

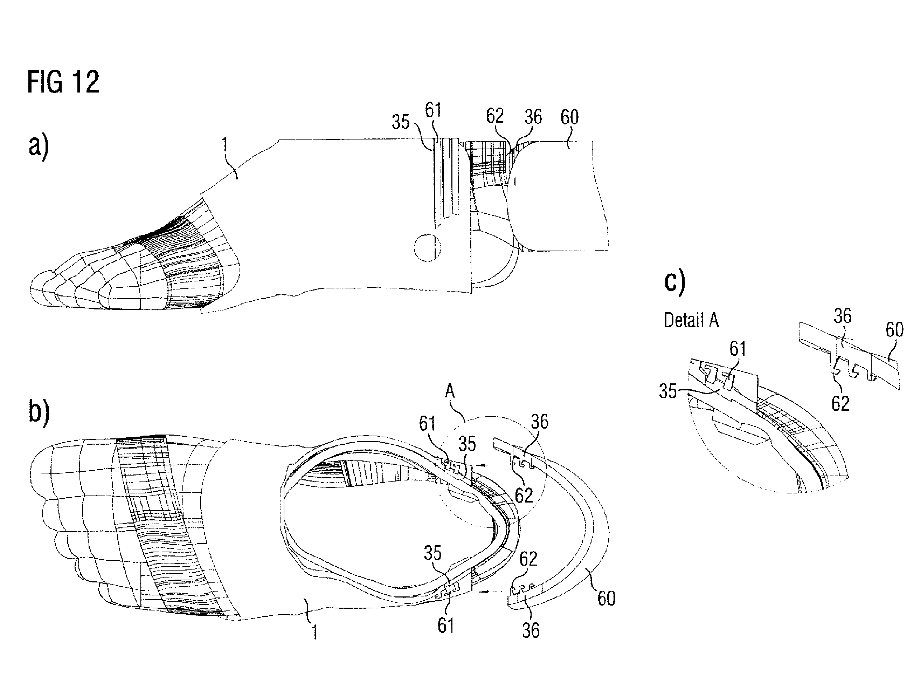

In Figur 12 ist ein als Ringfassung ausgebildetes erstes Schalenteil mit einer Fersenkappe in verschiedenen Ansichten zu sehen.

Figur 12 a) zeigt eine Ringfassung 1 zur Aufnahme eines linken Fußes mit einer Fersenkappe 60 in einer linken Seitenansicht in geöffnetem Zustand. Die Fersenkappe 60 ist mittels eines Ratschenverschlusses wie in Figur 9 dargestellt öffenbar und verschließbar bzw. einstellbar. Die Fersenkappe weist ferner die Form eines halb geöffneten breiten Ringes auf und ist an die Form der gedachten, umschlossenen Ferse angepasst. Wie in Figur 9 weist der Ratschenverschluss zwei Komponenten 35 und 36 auf, wobei die Komponente 35 links und rechts im Fersenbereich in die Außenfläche der Ringfassung 1 einlaminiert ist. Die hinterschnittenen Stege 61 der ersten Komponente 35 des Ratschenverschlusses stehen dabei aus der Außenfläche der Ringfassung heraus, wobei die Hinterschneidungen der Stege 61 in Richtung des Zehenbereichs der Ringfassung 1 zeigen. Die zweite Komponente 36 des Ratschenverschlusses ist im linken und rechten Bereich der Fersenkappe 60 in das Material der Innenfläche, also der der Orthese zugewandten Fläche, der Fersenkappe 60 einlaminiert. Die hinterschnittenen Stege 62 der zweiten Komponente 36 stehen dabei aus der Innenfläche heraus, sodass die Hinterschneidungen von der Orthese weg zeigen.

Zum Verschließen der Fersenkappe 60 werden die hinterschnittenen Stege 62 der zweiten Komponente 36 in die hinterschnittenen Stege 61 der ersten Komponente 35 eingehakt. Je nachdem wie dicht die Fersenkappe 60 an die Ringfassung 1 herangezogen werden soll, können mehr oder weniger hinterschnittene Stege 61 und 62 miteinander verhakt werden.

Figur 12 b) zeigt die Ringfassung 1 mit Fersenkappe 60 mit Ratschenverschluss in geöffnetem Zustand aus Figur 12 a) in einer Draufsicht. Im rechten Bildbereich sind die hakenförmigen Querschnitte der hinterschnittenen Stege 61 und 62 der Komponenten 35 und 36 des Ratschenverschlusses zu sehen.

Figur 12 c) zeigt eine Detailansicht einer Draufsicht auf die hinterschnittenen Stege 61 und 62 der Komponenten 35 und 36 des Ratschenverschlusses.

## Patentansprüche

1. Orthese zur Korrektur von Gelenkfehlstellungen mit einem ersten Schalenteil zur Aufnahme eines ersten Körperteils, einem zweiten Schalenteil zur Aufnahme eines zweiten mit dem ersten Körperteil durch ein Gelenk verbundenen zweiten Körperteils und mindestens einem Orthesengelenk mit zwei gegeneinander verdrehbaren Gelenkstücken
**dadurch gekennzeichnet, dass**
ein erstes Gelenkstück des Orthesengelenks an mindestens drei Befestigungspunkten auf einer durch die Befestigungspunkte definierten Befestigungsfläche des ersten Schalenteils um einen Drehpunkt des Orthesengelenks herum derart befestigt ist, dass der Drehpunkt des Orthesengelenks senkrecht beabstandet zur Befestigungsfläche liegt, und dass
die Orthese eine Schiene mit einem ersten und einem zweiten Teil aufweist, deren erster Teil als zweites Gelenkstück des Orthesengelenks ausgebildet ist und deren zweiter Teil am zweiten Schalenteil befestigt ist.

2. Orthese nach Anspruch 1, **dadurch gekennzeichnet, dass** die Orthese zwei bilateral angeordnete Orthesengelenke aufweist.

3. Orthese nach einem der vorhergehenden Ansprüche, **dadurch gekennzeichnet, dass** mindestens eines der Orthesengelenke ein Kugelgelenk oder ein Scharniergelenk ist.

4. Orthese nach dem vorhergehenden Anspruch, **dadurch gekennzeichnet, dass** mindestens eines der Orthesengelenke ein Kugelgelenk ist und zusätzlich zum ersten und zweiten Gelenkstück eine erste Anschlagscheibe zum Einschränken einer Bewegung des die Körperteile verbindenden Gelenks in eine erste Richtung, einen Innenring mit einer sphärisch und konvex gekrümmten Oberfläche und eine Abdeckscheibe aufweist, wobei das erste Gelenkstück als kreisförmige Scheibe mit einer senkrecht und zentral auf der Scheibe stehenden Gelenkachse ausgebildet ist, auf die Gelenkachse die erste Anschlagscheibe und dahinter der Innenring aufgeschoben ist, auf dem Innenring das zweite Gelenkstück gelagert ist und die Abdeckscheibe zum Verschließen und/oder Öffnen des Orthesengelenks an einem dem ersten Gelenkstück gegenüberliegenden Ende der Gelenkachse angeordnet ist.

5. Orthese nach dem vorhergehenden Anspruch, **dadurch gekennzeichnet, dass** das Orthesengelenk zwischen dem Innenring und der ersten Anschlagscheibe und/oder dem Innenring und der Abdeckscheibe eine zweite Anschlagscheibe zum Einschränken der Bewegung des die Körperteile verbindenden Gelenks in eine zweite Richtung aufweist.

6. Orthese nach einem der Ansprüche 4 oder 5, **dadurch gekennzeichnet, dass** der Innenring teilweise oder vollständig aus Teflon ist.

7. Orthese nach einem der Ansprüche 5 oder 6, **dadurch gekennzeichnet, dass** die erste und/oder die zweite Anschlagscheibe mittels Madenschrauben, Gewindestiften, Schrauben oder/und mittels sich auf dem ersten Gelenkstück sowie auf den Anschlagscheiben befindlichen Rasten einstellbar sind.

8. Orthese nach dem vorhergehenden Anspruch, **dadurch gekennzeichnet, dass** das Einstellen der Anschlagscheiben in 2°-Schritten, in 5°-Schritten und/oder in Schritten von Vielfachendes Abstands der Gewindestifte oder Schrauben möglich ist.

9. Orthese nach einem der vorhergehenden Ansprüche, **dadurch gekennzeichnet, dass** das zweite Gelenkstück Mittel zum Beschränken des Bewegungsbereichs des die Körperteile verbindenden Gelenks bzw. des Orthesengelenks in einer Richtung parallel zur Gelenkachse aufweist.

10. Orthese nach einem der vorhergehenden Ansprüche, **dadurch gekennzeichnet, dass** das erste Gelenkstück in dem ersten Schalenteil verankert, an das erste Schalenteil angeschraubt, angenietet oder in das erste Schalenteil einlaminiert ist.

11. Orthese nach einem der vorhergehenden Ansprüche, **dadurch gekennzeichnet, dass** die Position der Befestigungsfläche auf dem ersten Schalenteil variierbar ist und dadurch die Position des Drehpunkts des Orthesengelenks individuell positionierbar ist.
